# EUROPEAN PATENT APPLICATION

(11) **EP 4 571 310 A1**
(43) Date of publication of application: **18.06.2025**
(21) Application number: 23865311.7
(22) Date of filing: 31.08.2023
(51) Int. Cl.: G01N 33/53, C07K 16/18

(54) **P53 ISOFORM VARIANT FOR DIAGNOSING CANCER**

(30) Priority: 12.09.2022 JP 2022144861
(71) Applicant: Tuning Fork Bio, Inc., Cambridge, Massachusettes 02142 (US)
(72) Inventor: HIKICHI, Yuichi, Iwata-shi, Shizuoka 4388501 (JP); WATANABE, Shinya, Fukushima-shi, Fukushima 960-1295 (JP); TAKAGI, Motoki, Fukushima-shi, Fukushima 960-1295 (JP); IMAI, Junichi, Fukushima-shi, Fukushima 960-1295 (JP)
(74) Representative: Müller-Boré & Partner Patentanwälte PartG mbB
(86) International application number: PCT/JP2023/031842
(87) International publication number: WO 2024/057956

(57) **Abstract**

A method of collecting data for diagnosing cancer, determining an onset risk, determining a malignancy grade, and/or predicting a prognosis of cancer according to the present disclosure includes the steps of: (a) bringing a sample derived from a subject into contact with tumor-associated antigens to cause an antigen-antibody reaction; (b) measuring an amount of anti-p53 antibody, in the sample, that specifically binds to any of the tumor-associated antigens; and (c) comparing the measured amount of the anti-p53 antibody with a predetermined reference level of the anti-p53 antibody against the tumor-associated antigens, wherein the tumor-associated antigens include one or more p53 isoform variants consisting of amino acid sequences having a mutation in an amino acid sequence of any one of SEQ ID NOs: 1 to 3, and the mutation includes any one of an N-terminal deletion mutation, a C-terminal deletion mutation, and both the N-terminal deletion and the C-terminal deletion mutation.

## Description

### Technical Field

The present disclosure relates to a method of collecting data for diagnosing cancer, determining an onset risk, of cancer determining a malignancy grade of cancer, and/or predicting a prognosis of cancer by using p53 isoform variants, and a kit for diagnosing cancer.

### Background Art

TP53 is the most prominent gene in cancer research. Specifically, normal p53 protein is known to play an important role in, for instance, DNA repair, apoptosis induction, and cellular senescence for cancer suppression. However, it is considered that when some mutation occurs in TP53, a generated p53 protein variant may cause the formation of a malignant tumor. For example, it is believed that the p53 protein variant may promote cell proliferation and also cause cancer metastasis. It is also known that many isoforms exist in the translation product of TP53. Specifically, it is known that as isoforms of TP53, in addition to wild-type p53 isoform A, there are p53 isoforms B, C, G, I, H, D, E, F, J, K, and L having a difference in C-terminal structure due to alternative splicing or an N-terminal deletion mutation due to splicing abnormality (see Non Patent Literature 1 and Non Patent Literature 2). However, each isoform has many unclear points in its function.

On the other hand, in recent years, many methods using autoantibodies as markers of disease or disease sensitivity have been reported. For example, Patent Literature 1 describes a method by which when the level of an autoantibody that specifically binds to a tumor marker protein such as MUC1, MUC16, c-myc, EGFR, or p53 in a sample collected from a subject is increased, the subject can be identified as having an onset risk of cancer.

Further, as an auxiliary reagent for diagnosis of cancer to measure the expression level of an autoantibody (anti-p53 antibody) against p53 protein in human serum or plasma, "MESACUP (registered trademark) anti-p53 test", manufactured by MBL (MEDICAL & BIOLOGICAL LABORATORIES CO., LTD.), is known. Specifically, this reagent can be used to measure the expression level of anti-p53 antibody in serum or plasma by using wild-type p53 isoform A. The anti-p53 antibody appears with high specificity in malignant tumors, and there is little overlap in the positive rate for the type of cancer when used in combination with other cancer markers. Therefore, by combining the anti-p53 antibody and a plurality of other cancer markers to measure their values, the detection rate of a plurality of types of cancer such as esophageal cancer, colorectal cancer, and breast cancer can be efficiently improved.

### Citation List

### Patent Literature

Patent Literature 1: JP 2014-16364 A

### Non Patent Literature

Non Patent Literature 1: Jean-Christophe Bourdon et. al., "p53 isoforms can regulate p53 transcriptional activity", GENES & DEVELOPMENT, 2005, 19: 2122-2137
Non Patent Literature 2: V Marcel et. al., "Biological functions of p53 isoforms through evolution: lessons from animal and cellular models", Cell Death and Differentiation (2011) 18, 1815-1824

### Summary of Invention

The purpose of the present disclosure is to provide a method of collecting data for diagnosing cancer, determining an onset risk of cancer, determining a malignancy grade of cancer, and/or predicting a prognosis of cancer with good sensitivity while maintaining high specificity.

As a result of intensive studies to solve the above problems, the present inventors have arrived at the present disclosure.

A method according to a first aspect of the present disclosure is a method of collecting data for diagnosing cancer, determining an onset risk of cancer, determining a malignancy grade of cancer, and/or predicting a prognosis of cancer, the method including the steps of:
(a) bringing a sample derived from a subject into contact with one or more tumor-associated antigens to cause an antigen-antibody reaction;
(b) measuring an amount of anti-p53 antibody, in the sample, that specifically binds to any of the one or more tumor-associated antigens; and
(c) comparing the measured amount of the anti-p53 antibody with a predetermined reference level of the anti-p53 antibody against the one or more tumor-associated antigens,
wherein the tumor-associated antigens include one or more p53 isoform variants consisting of amino acid sequences having a mutation in an amino acid sequence of any one of SEQ ID NOs: 1 to 3, and the mutation includes any one of an N-terminal deletion mutation, a C-terminal deletion mutation, and both the N-terminal deletion mutation and the C-terminal deletion mutation.

A kit for diagnosing cancer according to a second aspect of the present disclosure includes one or more p53 isoform variants consisting of amino acid sequences having a mutation in an amino acid sequence of any one of SEQ ID NOs: 1 to 3, wherein the mutation includes any one of an N-terminal deletion mutation, a C-terminal deletion mutation, and both the N-terminal deletion mutation and the C-terminal deletion mutation.

### Brief Description of Drawings

FIG. 1 is a schematic diagram illustrating p53 isoforms A to L.
FIG. 2 is a schematic diagram showing the positions of nonsense mutations in respective p53 isoform variants.

### Description of Embodiments

As described above, use of each anti-p53 antibody in a sample as a cancer marker is useful because the anti-p53 antibody has high specificity for a malignant tumor. However, the anti-p53 antibody has high specificity for a plurality of types of cancer, but has a problem of low detection sensitivity for cancer. In other words, for example, when each anti-p53 antibody is used as a cancer marker and the marker is determined to be positive, there is a high possibility that the subject has developed cancer. However, even when it is determined to be negative, the possibility that the subject has developed cancer is not necessarily low. Hence, in general, when an anti-p53 antibody is used as a cancer marker, the antibody is often used in combination with other cancer markers, not only because there is little overlap in the positive rate with other cancer markers for the type of cancer, but also because the cancer detection sensitivity is low.

Therefore, when an anti-p53 antibody is used as a cancer marker, it is very useful if cancer can be detected with high sensitivity while maintaining high specificity peculiar to the anti-p53 antibody.

Currently, when an anti-p53 antibody is used as a cancer marker, wild-type p53 isoform A consisting of the amino acid sequence of SEQ ID NO: 1 is often used as an antigen for causing an antigen-antibody reaction. Further, as the kind of antibody isotype, an IgG antibody is generally used. The present inventors have conducted various studies on a method capable of detecting cancer with higher sensitivity while maintaining high specificity peculiar to an anti-p53 antibody as compared to this conventional typical method. Then, the present inventors have found findings described in the following (1) to (3), and completed the present disclosure.
(1) In human plasma, there are a plurality of anti-p53 IgG antibodies that specifically bind to respective p53 isoform variants and cannot be recognized by wild-type p53 isoform A. Further, there are significantly more of such specific anti-p53 antibodies against p53 isoform variants with any of an N-terminal deletion mutation, a C-terminal deletion mutation, and both the N-terminal and C-terminal deletion mutations. In other words, the anti-p53 antibodies are measured using respective p53 isoform variants having such a mutation and the results are then combined. This should make better the cancer detection sensitivity.
(2) In human plasma, there is an anti-p53 IgG antibody that specifically binds to p53 isoform C and cannot be recognized by wild-type p53 isoform A. In addition, there may be an anti-p53 IgG antibody that specifically binds to p53 isoform B. Thus, the anti-p53 antibodies are measured using p53 isoform B, p53 isoform C, and/or respective p53 isoform variants and the measurement results are then combined. This should make better the cancer detection sensitivity.
(3) In the results of assay for the anti-p53 antibodies that specifically bind to respective p53 isoform variants, the positive detection result by the IgG antibodies and the positive detection result by the IgA antibodies are different. Further, in the results of assay for the anti-p53 antibodies that specifically bind to p53 isoforms A to C, the positive detection result by the IgG antibodies and the positive detection result by the IgA antibodies are also different. Thus, the antibodies of an isotype other than currently commonly used IgG antibody are measured using p53 isoforms A to C and p53 isoform variants. This should make better the cancer detection sensitivity. Furthermore, it is assumed that the cancer detection sensitivity can be improved by combining anti-p53 antibody positive detection results by two or more antibodies (preferably two or more antibodies including an IgG antibody and an IgA antibody).

From the above findings (1) to (3), the following is understandable. A wide variety of anti-p53 antibody that could not be recognized by a conventional method can be detected by using, as each antigen for causing an antigen-antibody reaction, one or more of p53 isoform B, p53 isoform C, and p53 isoform variants having any one of an N-terminal deletion mutation (p53 isoform G, I, H, D, E, F, J, K or L), a C-terminal deletion mutation, and both the N-terminal and C-terminal deletion mutations. As a result, it is assumed that cancer can be detected with higher sensitivity. In addition, it is also assumed that cancer can be detected with higher sensitivity by using wild-type p53 isoform A in combination as an antigen.

Also, as an antigen, one or more p53 isoforms A to C and p53 isoform variants having the above-described deletion mutation are used to detect antibodies of one or more isotypes other than an IgG antibody or antibodies of two or more isotypes including an IgG antibody among all isotypes. Thus, it is also assumed that cancer can be detected with higher sensitivity.

As described above, by utilizing the findings of the above (1) to (3), it is possible to implement a method of collecting various data for diagnosing cancer, determining an onset risk of cancer, determining a malignancy grade of cancer, and/or predicting a prognosis of cancer with good sensitivity while maintaining high specificity peculiar to the anti-p53 antibody as a cancer marker.

That is, the present disclosure can provide a method of collecting data for diagnosing cancer, determining an onset risk of cancer, determining a malignancy grade of cancer, and/or predicting a prognosis of cancer with good sensitivity while maintaining high specificity.

### <Definitions>

As used herein, the "wild-type p53 isoform A" mainly means a protein consisting of the amino acid sequence of SEQ ID NO: 1. However, the "wild-type p53 isoform A" may contain some mutations such as addition, deletion and/or substitution of amino acids as described later as long as the specific binding to the anti-p53 antibody is at the level equivalent to that for the protein of SEQ ID NO: 1.

As used herein, the "p53 isoform B" mainly means a protein consisting of the amino acid sequence of SEQ ID NO: 2. However, the "p53 isoform B" may contain some mutations such as addition, deletion and/or substitution of amino acids as described later as long as the specific binding to the anti-p53 antibody is at the level equivalent to that for the protein of SEQ ID NO: 2. As used herein, the "p53 isoform C" mainly means a protein consisting of the amino acid sequence of SEQ ID NO: 3. However, the "p53 isoform C" may contain some mutations such as addition, deletion and/or substitution of amino acids as described later as long as the specific binding to the anti-p53 antibody is at the level equivalent to that for the protein of SEQ ID NO: 3. Note that the p53 isoform B and the p53 isoform C are each an isoform having a difference in its C-terminal structure due to alternative splicing as compared with the wild-type p53 isoform A.

As used herein, the "p53 isoform variant having an N-terminal deletion mutation" means a p53 isoform variant consisting of an amino acid sequence having an N-terminal deletion mutation due to a splicing abnormality in the amino acid sequence of one of SEQ ID NOs: 1 to 3. However, the p53 isoform variant having an N-terminal deletion mutation may contain mutations other than deletion mutations, such as addition, deletion and/or substitution of amino acids as described later as long as the specific binding to the anti-p53 antibody is not affected. Specific examples of such a p53 isoform variant having an N-terminal deletion mutation include p53 isoform G (SEQ ID NO: 4), p53 isoform I (SEQ ID NO: 5), p53 isoform H (SEQ ID NO: 6), p53 isoform D (SEQ ID NO: 7), p53 isoform E (SEQ ID NO: 8), p53 isoform F (SEQ ID NO: 9), p53 isoform J (SEQ ID NO: 10), p53 isoform K (SEQ ID NO: 11), or p53 isoform L (SEQ ID NO: 12) (see Non Patent Literature 1 and Non Patent Literature 2).

As used herein, the "p53 isoform variant having a C-terminal deletion mutation" means a p53 isoform variant consisting of an amino acid sequence having a C-terminal deletion mutation due to a nonsense mutation in the amino acid sequence of one of SEQ ID NOs: 1 to 3. However, the p53 isoform variant having a C-terminal deletion mutation may contain mutations other than deletion mutations, such as addition, deletion and/or substitution of amino acids as described later as long as the specific binding to the anti-p53 antibody is not affected. Specific examples of such a p53 isoform variant having a C-terminal deletion mutation include a protein consisting of the amino acid sequence of one of SEQ ID NOs: 13 to 18.

As used herein, the "p53 isoform variant having both N-terminal and C-terminal deletion mutations" means a p53 isoform variant consisting of an amino acid sequence having an N-terminal deletion mutation due to a splicing abnormality and a C-terminal deletion mutation due to a nonsense mutation in the amino acid sequence of one of SEQ ID NOs: 1 to 3. However, the p53 isoform variant having both N-terminal and C-terminal deletion mutations may contain mutations other than deletion mutations, such as addition, deletion and/or substitution of amino acids as described later as long as the specific binding to the anti-p53 antibody is not affected. Specifically, the "p53 isoform variant having both N-terminal and C-terminal deletion mutations" means a p53 isoform variant consisting of an amino acid sequence having a C-terminal deletion mutation due to a nonsense mutation in the amino acid sequence of one of SEQ ID NOs: 4 to 6, 7 to 9, or 10 to 12. Examples of such a p53 isoform variant having both N-terminal and C-terminal deletion mutations include a protein consisting of the amino acid sequence of one of SEQ ID NOs: 19 to 35.

As used herein, the term "anti-p53 antibody (or autoantibody against p53 protein)" means an antibody that is produced in the body of a subject of interest and targets the p53 protein present in the body of the subject.

As used herein, the wording "diagnosing cancer, determining an onset risk of cancer, determining a malignancy grade of cancer, and/or predicting a prognosis of cancer" means to include: determining or predicting whether a subject is likely to have cancer; determining or predicting the onset risk of cancer in a subject; determining or predicting the degree of malignancy, severity, or progression of cancer in a subject; monitoring (e.g., continuously) the therapeutic effect of cancer in a subject; and/or determining or predicting a poor prognosis of cancer in a subject.

As used herein, the term "cancer" is not particularly limited, and examples thereof include brain tumor, head and neck cancer, breast cancer, uterine body cancer, cervical cancer, uterine sarcoma, ovarian cancer, esophageal cancer, salivary gland cancer, thyroid cancer, stomach cancer, appendix cancer, large bowel cancer, liver cancer, gallbladder cancer, bile duct cancer, pancreatic cancer, adrenal cancer, gastrointestinal stromal tumor (GIST), mesothelioma, kidney cancer, small intestine cancer, lung cancer, small cell lung cancer, renal pelvis and ureter cancer, osteosarcoma, Ewing's sarcoma, chondrosarcoma, prostate cancer, testicular tumor, renal cell cancer, bladder cancer, rhabdomyosarcoma, skin cancer, anal cancer, mesothelioma, malignant lymphoma, leukemia, chronic lymphocytic leukemia (CLL), multiple myeloma, or melanoma.

As used herein, the "subject" mainly means humans and other mammals. Examples of other mammals include primate animals (e.g., monkeys, chimpanzees), livestock animals (e.g., cows, horses, pigs, sheep), pet animals (e.g., dogs cats), or experimental animals (e.g., mice, rats, rabbits). Among them, the subject is preferably a human.

As used herein, the "sample (or sample derived from a subject)" is not particularly limited as long as the sample is derived from a subject and is suitable for collecting data for diagnosing cancer, determining an onset risk of cancer, determining a malignancy grade of cancer, and/or predicting a prognosis of cancer, and is capable of causing an antigen-antibody reaction by being brought into contact with an antigen. In addition, as used herein, the "sample (or sample derived from a subject)" also includes a post-preparation sample obtained by preparing, with a diluent or the like, for instance, a body fluid collected directly from a subject. Further, it is preferable to select a suitable sample according to the isotype of the antibody to be measured. Examples of the sample include whole blood, serum, plasma, urine, prostate fluid, tears, mucoascites, oral fluid, saliva, semen, seminal plasma, mucus, sweat, nasal mucus, vaginal fluid, feces, sputum, cerebrospinal fluid, bone marrow, lymph fluid, fetal fluid (e.g., amniotic fluid), or milk. From the viewpoint of ease of collection, the sample preferably contains one or more of serum, plasma, oral fluid, and saliva, and more preferably contains one or more of serum and plasma.

As used herein, the term "positive" means that the subject is highly likely to be cancer, the onset risk of cancer in the subject is high, the degree of malignancy, severity or progression of cancer in the subject is high, the cancer therapeutic effect on the subject is low, and others since the amount of the anti-p53 antibody as a cancer marker in a sample is larger than a predetermined reference level. As used herein, the term "negative" means that the subject is less likely to be cancer, the onset risk of cancer in the subject is low, the degree of malignancy, severity or progression of cancer in the subject is low, the cancer therapeutic effect on the subject is high, and others since the amount of the anti-p53 antibody as a cancer marker in a sample is less than a predetermined reference level.

Hereinafter, embodiments of the present disclosure will be described in detail. Note that the scope of the present disclosure is not limited to the embodiments described herein, and various modifications can be made without departing from the spirit of the present disclosure.

### <Embodiment 1: Method of collecting data for diagnosing cancer, determining onset risk of cancer, determining malignancy grade of cancer, and/or predicting prognosis of cancer>

The method in Embodiment 1 includes four methods. Specifically, included are: a method related to utilization of p53 isoform variants with a deletion mutation(s) (Embodiment 1-1); a method related to utilization of any one of p53 isoform B, p53 isoform C, and p53 isoform variants with a deletion mutation(s) (Embodiment 1-2); a method related to an assay for antibodies other than IgG antibodies (Embodiment 1-3); and a method related to an assay for antibodies of two or more isotypes (Embodiment 1-4). Details will be described below.

### (Embodiment 1-1)

The method in Embodiment 1-1 is a method including using, as a cancer marker, an anti-p53 antibody that specifically binds to a p53 isoform variant having a deletion mutation. Specifically, the method in Embodiment 1-1 includes (a) an antigen-antibody reaction step, (b) a step of measuring an amount of anti-p53 antibody, and (c) a step of comparing the amount of the anti-p53 antibody. Each step will be described in detail below.

### (a) Antigen-antibody reaction step

In the antigen-antibody reaction step, an antigen-antibody reaction is caused by bringing a sample derived from a subject into contact with one or more tumor-associated antigens.

First, the following details the tumor-associated antigens to be brought into contact with a sample.

The tumor-associated antigens include any one or more of p53 isoform variants with an N-terminal deletion mutation, p53 isoform variants with a C-terminal deletion mutation, and p53 isoform variants with both a N-terminal deletion mutation and a C-terminal deletion mutation (hereinafter, these variants are also referred to as "p53 isoform variants of Embodiment 1-1").

The p53 isoform variants of Embodiment 1-1 are each preferably a protein represented by any one of the following (i) to (iii).
(i) A protein consisting of the amino acid sequence of any one of SEQ ID NOs: 4 to 35.
(ii) A protein consisting of an amino acid sequence having one or several amino acids added, deleted and/or substituted in the amino acid sequence of any one of SEQ ID NOs: 4 to 35 and capable of specifically binding to an anti-p53 antibody specifically binding to the protein of the corresponding SEQ ID NO described in (i) at a level equivalent to that for the protein of the corresponding SEQ ID NO.
(iii) A protein consisting of an amino acid sequence having a homology of 95% or more to the amino acid sequence of any one of SEQ ID NOs: 4 to 35 and capable of specifically binding to an anti-p53 antibody specifically binding to the protein of the corresponding SEQ ID NO described in (i) at a level equivalent to that for the protein of the corresponding SEQ ID NO.

In the p53 isoform variants of Embodiment 1-1, the protein consisting of the amino acid sequence of any one of SEQ ID NOs: 4 to 35 in the above (i) is a p53 isoform variant having any one of the N-terminal deletion mutation, the C-terminal deletion mutation, and both the N-terminal and C-terminal deletion mutations in the amino acid sequence of one of SEQ ID NOs: 1 to 3. Table 1 below collectively provides the amino acid sequence SEQ ID NOs of the proteins (including the wild-type p53 isoform A, the p53 isoform B, and the p53 isoform C) of the above (i), the nucleotide sequence SEQ ID NOs, the specific mutations in the wild-type p53 isoform A, and so on.

**[Table 1]**

| Amino acid sequence SEQ ID NO | Nucleotide sequence SEQ ID NO | Isoform type | Kind of nonsense mutation | Specific mutation in WT amino acid sequence |
|---|---|---|---|---|
| 1 | 36 | A(p53α) | - | - |
| 2 | 37 | B(p53β) | - | Difference in C-terminal structure (Alternative splicing) |
| 3 | 38 | C(p53γ) | - | |
| 4 | 39 | G(Δ40p53α) | - | N-terminal deletion (Splicing abnormality) |
| 5 | 40 | I(Δ40p53β) | - | |
| 6 | 41 | H(Δ40p53γ) | - | |
| 7 | 42 | D(Δ133p53α) | - | |
| 8 | 43 | E(Δ133p53β) | - | |
| 9 | 44 | F(Δ133p53γ) | - | |
| 10 | 45 | J(Δ160p53α) | - | |
| 11 | 46 | K(Δ160p53β) | - | |
| 12 | 47 | L(Δ160p53γ) | - | |
| 13 | 48 | p53(A(p53α)) | R342X | C-terminal deletion (Nonsense mutation) |
| 14 | 49 | p53 | Q331X | |
| 15 | 50 | p53 | Q317X | |
| 16 | 51 | p53 | R306X | |
| 17 | 52 | p53 | Q192X | |
| 18 | 53 | p53 | W146X | |
| 19 | 54 | Δ40p53(G(Δ40p53α)) | R303X | N- and C-terminal deletions (Splicing abnormality and nonsense mutation) |
| 20 | 55 | Δ40p53 | Q292X | |
| 21 | 56 | Δ40p53 | Q278X | |
| 22 | 57 | Δ40p53 | R267X | |
| 23 | 58 | Δ40p53 | Q153X | |
| 24 | 59 | Δ40p53 | W107X | |
| 25 | 60 | Δ133p53(D(Δ133p53α)) | R210X | |
| 26 | 61 | Δ133p53 | Q199X | |
| 27 | 62 | Δ133p53 | Q185X | |
| 28 | 63 | Δ133p53 | R174X | |
| 29 | 64 | Δ133p53 | Q60X | |
| 30 | 65 | Δ133p53 | W14X | |
| 31 | 66 | Δ160p53(J(Δ160p53α)) | R183X | |
| 32 | 67 | Δ160p53 | Q172X | |
| 33 | 68 | Δ160p53 | Q158X | |
| 34 | 69 | Δ160p53 | R147X | |
| 35 | 70 | Δ160p53 | Q33X | |

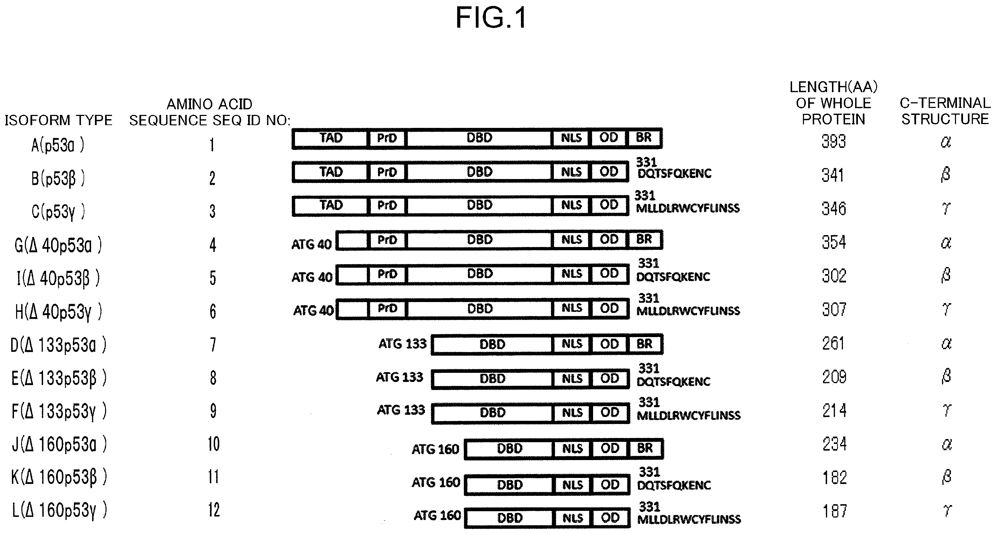

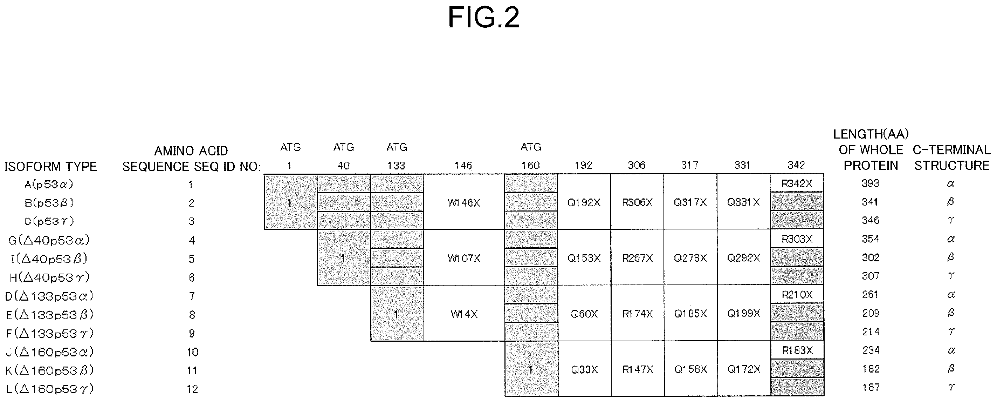

In addition, FIG. 1 schematically shows the structures of wild-type p53 isoforms A and p53 isoforms B, C, G, I, H, D, E, F, J, K and L (see also Non Patent Literatures 1 and 2). Further, FIG. 2 schematically illustrates the positions of nonsense mutations of respective p53 isoform variants on the sequences of wild-type p53 isoform A and p53 isoforms B, C, G, I, H, D, E, F, J, K and L.

As shown in FIG. 1, the p53 isoform B (SEQ ID NO: 2) and the p53 isoform C (SEQ ID NO: 3) differ from the wild-type p53 isoform A in the amino acid sequence in the C-terminal structure. The p53 isoforms G (SEQ ID NO: 4), I (SEQ ID NO: 5), H (SEQ ID NO: 6), D (SEQ ID NO: 7), E (SEQ ID NO: 8), F (SEQ ID NO: 9), J (SEQ ID NO: 10), K (SEQ ID NO: 11) and L (SEQ ID NO: 12) each consist of an amino acid sequence having only an N-terminal deletion mutation in the amino acid sequence of one of the p53 isoforms A to C.

Further, as shown in Table 1 above, examples of the p53 isoform variant having only a C-terminal deletion mutation include a p53 isoform variant consisting of the amino acid sequence of SEQ ID NO: 13 or SEQ ID NO: 14. As shown in FIG. 2, the p53 isoform variant consisting of the amino acid sequence of SEQ ID NO: 13 is a protein having a C-terminal deletion mutation, in which arginine at position 342 was substituted with a stop codon due to a nonsense mutation in the amino acid sequence of SEQ ID NO: 1 (in FIG. 2, the mutation is indicated as "R342X"). In addition, as shown in FIG. 2, the p53 isoform variant consisting of the amino acid sequence of SEQ ID NO: 14 is a protein having a C-terminal deletion mutation, in which glutamine at position 331 is substituted with a stop codon due to a nonsense mutation in the amino acid sequence of any one of SEQ ID NOs: 1 to 3 (in FIG. 2, the mutation is indicated as "Q331X").

In addition, as shown in Table 1 above, examples of the p53 isoform variant having both an N-terminal deletion mutation and a C-terminal deletion mutation include a p53 isoform variant consisting of the amino acid sequence of SEQ ID NO: 19 or SEQ ID NO: 20. As shown in FIG. 2, the p53 isoform variant consisting of the amino acid sequence of SEQ ID NO: 19 is a protein having both an N-terminal deletion mutation and a C-terminal deletion mutation, in which arginine at position 303 is substituted with a stop codon due to a nonsense mutation in the amino acid sequence of SEQ ID NO: 4 having only an N-terminal deletion mutation (in FIG. 2, the mutation is indicated as "R303X"). In addition, as shown in FIG. 2, the p53 isoform variant consisting of the amino acid sequence of SEQ ID NO: 20 is a protein having both an N-terminal deletion mutation and a C-terminal deletion mutation, in which glutamine at position 292 is substituted with a stop codon due to a nonsense mutation in the amino acid sequence of any one of SEQ ID NOs: 4 to 6 having only an N-terminal deletion mutation (in FIG. 2, the mutation is indicated as "Q292X").

As described in (ii) and (iii) above, the amino acid sequence of the p53 isoform variants of Embodiment 1-1 may not be exactly the same as any one of SEQ ID NOs: 4 to 35.

As used herein, the wording "capable of specifically binding to an anti-p53 antibody specifically binding to the protein of the corresponding SEQ ID NO (or a specific SEQ ID NO) at a level equivalent to that for the protein of the corresponding SEQ ID NO" means (and is characterized in), for example, as will be described in detail in the following Examples, that when the amount of the anti-p53 antibody in the same sample is measured with a protein microarray, the value after normalization is substantially the same as that of the protein of the corresponding SEQ ID NO.

Further, as used herein, the term "one or several" means, but is not particularly limited to, for example, 1 to 20, 1 to 15, 1 to 10, 1 to 7, 1 to 5, 1 to 4, 1 to 3, or 1 to 2.

As used herein, the "amino acid sequence having a homology of a specific value or more" is not particularly limited, but is intended to be, for example, an amino acid sequence having a homology of a specific value or more calculated using a technique such as BLAST known to those skilled in the art. Furthermore, as used herein, the "amino acid sequence having a homology of 95% or more" is an amino acid sequence having a homology of preferably 96% or more, more preferably 97% or more, still more preferably 98% or more, and particularly preferably 99% or more.

In the method of Embodiment 1-1, any one or more of the p53 isoform variants of Embodiment 1-1 in the above (i) to (iii) (preferably (i)) can be used as a tumor-associated antigen(s) for measuring an anti-p53 antibody as a cancer marker. This makes it possible to detect an anti-p53 antibody in a sample, which antibody could not be recognized by the conventional method. Thus, data for diagnosing cancer or the like can be collected while the sensitivity is favorable and high specificity is maintained.

The p53 isoform variants of Embodiment 1-1 are each more preferably a protein represented by any one of the following (I) to (III) in view of the results of Examples by the present inventors.
(I) A protein consisting of the amino acid sequence of any one of SEQ ID NOs: 4 to 6, 7, 8, 10, 11, 14, 16 to 24, 26, and 29 to 35.
(II) A protein consisting of an amino acid sequence having one or several amino acids added, deleted and/or substituted in the amino acid sequence of any one of SEQ ID NOs: 4 to 6, 7, 8, 10, 11, 14, 16 to 24, 26, and 29 to 35 and capable of specifically binding to an anti-p53 antibody specifically binding to the protein of the corresponding SEQ ID NO described in (I) at a level equivalent to that for the protein of the corresponding SEQ ID NO.
(III) A protein consisting of an amino acid sequence having a homology of 95% or more to the amino acid sequence of any one of SEQ ID NOs: 4 to 6, 7, 8, 10, 11, 14, 16 to 24, 26, and 29 to 35 and capable of specifically binding to an anti-p53 antibody specifically binding to the protein of the corresponding SEQ ID NO described in (I) at a level equivalent to that for the protein of the corresponding SEQ ID NO.

In the method of Embodiment 1-1, any one or more of the p53 isoform variants of Embodiment 1-1 in the above (I) to (III) (preferably (I)) can be used as a tumor-associated antigen(s) for measuring an anti-p53 antibody as a cancer marker. This makes it possible to more reliably detect an anti-p53 antibody in a sample, which antibody could not be recognized by the conventional method. Thus, data for diagnosing cancer or the like can be collected more reliably while the sensitivity is favorable and high specificity is maintained.

Further, the p53 isoform variants of Embodiment 1-1 as tumor-associated antigens are preferably used in combination of two or more thereof. Specifically, it is preferable that a sample derived from the same subject is brought into contact with each of two or more p53 isoform variants of Embodiment 1-1 to cause each antigen-antibody reaction. In this case, steps (a) to (c), including steps (b) to (c) described below, are preferably carried out using each of the p53 isoform variants of Embodiment 1-1 as tumor-associated antigens.

Use of two or more kinds of the p53 isoform variants of Embodiment 1-1 as tumor-associated antigens in combination makes it possible to widely detect an anti-p53 antibody that specifically binds to each p53 isoform variant. Note that the p53 isoform variants of Embodiment 1-1 as tumor-associated antigens are more preferably used in combination of 3 or more, still more preferably used in combination of 4 or more, and particularly preferably used in combination of 5 or more.

In addition, the tumor-associated antigens preferably further include one or more p53 isoforms each consisting of the amino acid sequence of any one of SEQ ID NOs: 1 to 3. That is, in addition to the p53 isoform variants of Embodiment 1-1, it is preferable to use one or more of wild-type p53 isoform A, p53 isoform B, and p53 isoform C in combination as the tumor-associated antigens. Also in this case, a sample derived from the same subject is brought into contact with the p53 isoform variants of Embodiment 1-1 and respective p53 isoforms to cause each antigen-antibody reaction. Further, steps (a) to (c), including steps (b) to (c) described below, are preferably carried out using each of the p53 isoform variants of Embodiment 1-1 and respective p53 isoforms.

As a method for causing an antigen-antibody reaction, any method known to those skilled in the art can be applied as long as an antigen-antibody reaction can be caused by bringing a sample, containing anti-p53 antibody, derived from a subject into contact with tumor-associated antigens. Specifically, for example, any immunoassay known to those skilled in the art can be applied together with the following step (b) of measuring the amount of the anti-p53 antibody.

### (b) Step of measuring amount of anti-p53 antibody

The step of measuring the amount of anti-p53 antibody includes measuring the amount of each anti-p53 antibody in a sample, which antibody specifically binds to one or more tumor-associated antigens.

That is, in this step, the amount of each anti-p53 antibody as a cancer marker in the sample, which antibody specifically binds to any of the p53 isoform variants of Embodiment 1-1, is measured.

When two or more p53 isoform variants of Embodiment 1-1 are used in combination as the tumor-associated antigens, the amount of each anti-p53 antibody in the sample, which antibody specifically binds to any of the p53 isoform variants of Embodiment 1-1, is measured. In addition, when not only the p53 isoform variants of Embodiment 1-1 but also one or more of wild-type p53 isoforms A, B, and C are used in combination as the tumor-associated antigens, the amount of each anti-p53 antibody in the sample, which antibody specifically binds to any of the p53 isoform variants of Embodiment 1-1 and the amount of each anti-p53 antibody in the sample, which antibody specifically binds to the corresponding p53 isoform, are measured.

As used herein, the term "measuring (or assay)" means to measure the amount (or expression level) or concentration of each anti-p53 antibody in the sample derived from the subject, preferably semi-quantitatively or quantitatively. The amount may be an absolute amount or an amount relative to the amount in a negative subject.

Further, the isotype of the anti-p53 antibody preferably includes one or more isotypes selected from the group consisting of IgG, IgA, IgM, and IgE antibodies, more preferably two or more isotypes thereof, and still more preferably three or more isotypes thereof. In addition, the isotype of the anti-p53 antibody includes more preferably one or more of an IgG antibody and an IgA antibody, and still more preferably a combination of an IgG antibody and an IgA antibody.

When the isotype of the anti-p53 antibody includes two or more isotypes, steps (b) to (c), including step (c) described later, are preferably performed using each isotype of the anti-p53 antibody. It is assumed that the cancer detection sensitivity can be more efficiently improved after the subsequent step (c) by combining the results from the measured amounts of two or more anti-p53 antibody isotypes.

How to measure the amount of each anti-p53 antibody as a cancer marker is not particularly limited, and any method known to those skilled in the art can be applied. For example, as the assay method, any immunoassay known to those skilled in the art can be applied, such as an ELISA (Enzyme Linked Immunosorbent Assay), Western blotting, flow cytometry, a multiplex assay with, for instance, a Luminex (registered trademark), U-Plex, or Focus array, or surface plasmon resonance.

### (c) Step of comparing amount of anti-p53 antibody

The step of comparing the amount of each anti-p53 antibody includes comparing the measured amount of each anti-p53 antibody with a predetermined reference level of the anti-p53 antibody against one or more kinds of tumor-associated antigens.

As used herein, the "predetermined reference level of the anti-p53 antibody against the tumor-associated antigen" means an anti-p53 antibody level (or concentration, normalized value, or the like) that is defined in advance according to, for instance, an assay method, a type of a sample, and/or a type of cancer, and serves as an index such that the subject is likely to have cancer, the onset risk of cancer in the subject is high, the cancer malignancy grade of the subject is high, and so on. Alternatively, the "predetermined reference level of the anti-p53 antibody against the tumor-associated antigen" may mean an anti-p53 antibody level (or concentration, normalized value, or the like) that is defined in advance according to, for instance, an assay method, a type of a sample, and/or a type of cancer, and serves as an index such that the subject is less likely to have cancer, the onset risk of cancer in the subject is low, the cancer malignancy grade of the subject is low, and so on.

Such a reference level can be determined, for example, based on an amount derived from a cancer patient population, as measured by exactly the same method. The cancer patient population may be, for example, a population diagnosed as cancer by a physician or a population determined to have a high onset risk of cancer by using a plurality of other cancer markers. Alternatively, such a reference level can be determined, for example, based on an amount derived from a healthy subject population, as measured by exactly the same method. The cancer patient population may be, for example, a population not diagnosed as cancer by a physician or a population determined to have a low onset risk of cancer by using a plurality of other cancer markers. The reference level may vary depending on various physiological parameters such as the type, age, gender, etc. of the subject.

That is, this step includes comparing the measured amount of the anti-p53 antibody with a reference level that can be an index of cancer to collect data for diagnosing cancer, determining an onset risk of cancer, determining a malignancy grade of cancer, and/or predicting a prognosis of cancer.

When two or more p53 isoform variants of Embodiment 1-1 are used in combination as the tumor-associated antigens, the measured amount of each anti-p53 antibody against the p53 isoform variant(s) of Embodiment 1-1 is compared to the reference level. As a result, it is assumed that the cancer detection sensitivity can be efficiently improved by combining the determination results based on the measured amount of each anti-p53 antibody widely detected using the p53 isoform variants of Embodiment 1-1.

Further, when not only the p53 isoform variants of Embodiment 1-1 but also one or more of wild-type p53 isoforms A, B, and C are used in combination as the tumor-associated antigens, the measured amount of each anti-p53 antibody is compared to each corresponding reference level. As a result, it is assumed that the cancer detection sensitivity can be more efficiently improved by combining the determination results based on the measured amount of each anti-p53 antibody widely detected using the p53 isoform variants of Embodiment 1-1 and respective p53 isoforms.

When two or more anti-p53 antibody isotypes are measured, the measured amount of each anti-p53 antibody isotype is compared with each corresponding reference level. As a result, it is assumed that the cancer detection sensitivity can be more efficiently improved by combining the determination results using the respective isotypes.

As such, the method of Embodiment 1-1 makes it possible to collect data for diagnosing cancer, determining an onset risk of cancer, determining a malignancy grade of cancer, and/or predicting a prognosis of cancer with good sensitivity while maintaining high specificity peculiar to the anti-p53 antibody.

### (Embodiment 1-2)

A method in Embodiment 1-2 is a method including using, as a cancer marker, an anti-p53 antibody that specifically binds to one or more of p53 isoform B, p53 isoform C, and p53 isoform variants of Embodiment 1-1.

As described above, when an anti-p53 antibody is used as a cancer marker, wild-type p53 isoform A consisting of the amino acid sequence of SEQ ID NO: 1 is used in the conventional method.

However, according to the findings found by the present inventors, it is assumed that an anti-p53 IgG antibody that specifically binds to p53 isoform B (SEQ ID NO: 2) or p53 isoform C (SEQ ID NO: 3) and cannot be recognized by wild-type p53 isoform A may also be present. Therefore, the method in Embodiment 1-2 described below allows for detection of the anti-p53 antibody in a sample, which antibody cannot be recognized by the conventional method. This thus makes it possible to collect data for diagnosing cancer, determining an onset risk of cancer, determining a malignancy grade of cancer, and/or predicting a prognosis of cancer with good sensitivity while maintaining high specificity.

The method in Embodiment 1-2 is a method of collecting data for diagnosing cancer, determining an onset risk of cancer, determining a malignancy grade of cancer, and/or predicting a prognosis of cancer, the method including the steps of:
(a) bringing a sample derived from a subject into contact with one or more tumor-associated antigens to cause an antigen-antibody reaction;
(b) measuring an amount of anti-p53 antibody, in the sample, that specifically binds to any of the one or more tumor-associated antigens; and
(c) comparing the measured amount of the anti-p53 antibody with a predetermined reference level of the anti-p53 antibody against the one or more tumor-associated antigens,
wherein the tumor-associated antigens include one or more proteins selected from the group consisting of p53 isoform B, p53 isoform C, and p53 isoform variants of Embodiment 1-1.

The p53 isoform B of Embodiment 1-2 may be a protein represented by any one of the following (i') to (iii').
(i') A protein consisting of the amino acid sequence of SEQ ID NO: 2.
(ii') A protein consisting of an amino acid sequence having one or several amino acids added, deleted and/or substituted in the amino acid sequence of SEQ ID NO: 2 and capable of specifically binding to an anti-p53 antibody specifically binding to the protein of SEQ ID NO: 2 at a level equivalent to that for the protein of SEQ ID NO: 2.
(iii') A protein consisting of an amino acid sequence having a homology of 95% or more to the amino acid sequence of SEQ ID NO: 2 and capable of specifically binding to an anti-p53 antibody specifically binding to the protein of SEQ ID NO: 2 at a level equivalent to that for the protein of SEQ ID NO 2.

The p53 isoform C of Embodiment 1-2 may be a protein represented by any one of the following (i") to (iii").
(i") A protein consisting of the amino acid sequence of SEQ ID NO: 3.
(ii") A protein consisting of an amino acid sequence having one or several amino acids added, deleted and/or substituted in the amino acid sequence of SEQ ID NO: 3 and capable of specifically binding to an anti-p53 antibody specifically binding to the protein of SEQ ID NO: 3 at a level equivalent to that for the protein of SEQ ID NO: 3.
(iii") A protein consisting of an amino acid sequence having a homology of 95% or more to the amino acid sequence of SEQ ID NO: 3 and capable of specifically binding to an anti-p53 antibody specifically binding to the protein of SEQ ID NO: 3 at a level equivalent to that for the protein of SEQ ID NO 3.

That is, in the method of Embodiment 1-2, any one or more of p53 isoform B, p53 isoform C, and the p53 isoform variants of Embodiment 1-1 are each used as a tumor-associated antigen for measuring an anti-p53 antibody as a cancer marker. Except for the above, the other configurations are the same as those of Embodiment 1-1 described above. Specifically, the definition of each term, the details of each step, the preferred embodiments, and the like are the same as those in Embodiment 1-1 described above.

For example, similar to Embodiment 1-1 described above, it is preferable to use two or more kinds among the p53 isoform B, the p53 isoform C, and the p53 isoform variants of Embodiment 1-1 in combination as the tumor-associated antigens. For example, similar to Embodiment 1-1 described above, it is possible to use not only one or more kinds among the p53 isoform B, the p53 isoform C, and the p53 isoform variants of Embodiment 1-1, but also the wild-type p53 isoform A consisting of the amino acid sequence of SEQ ID NO: 1 in combination as the tumor-associated antigens. Further, for example, similar to Embodiment 1-1 described above, the isotype of the anti-p53 antibody is preferably one or more types selected from the group consisting of IgG, IgA, IgM, and IgE antibodies, and when there are two or more isotypes of the anti-p53 antibody, steps (b) to (c) are preferably performed on each anti-p53 antibody isotype.

### (Embodiment 1-3)

The method in Embodiment 1-3 is a method including measuring an anti-p53 antibody of an isotype other than an IgG antibody to collect data for diagnosing cancer, determining an onset risk of cancer, determining a malignancy grade of cancer, and/or predicting a prognosis of cancer.

As described above, as the kind of antibody isotype, an IgG antibody is generally used in the conventional method.

However, according to the findings found by the present inventors, it is assumed that the different kinds of the antibody isotype cause different cancer detection results by the amount of anti-p53 antibody that specifically binds to any one of proteins among p53 isoform A, p53 isoform B, p53 isoform C, and p53 isoform variants of Embodiment 1-1. Therefore, the method in Embodiment 1-3 described below allows for detection of a wide variety of the anti-p53 antibody in a sample, which antibody cannot be recognized by the conventional method. This thus makes it possible to collect data for diagnosing cancer, determining an onset risk of cancer, determining a malignancy grade of cancer, and/or predicting a prognosis of cancer with good sensitivity while maintaining high specificity.

A method in Embodiment 1-3 is a method of collecting data for diagnosing cancer, determining an onset risk of cancer, determining a malignancy grade of cancer, and/or predicting a prognosis of cancer, the method including the steps of:
(a) bringing a sample derived from a subject into contact with one or more tumor-associated antigens to cause an antigen-antibody reaction;
(b) measuring an amount of anti-p53 antibody, in the sample, that specifically binds to any of the one or more tumor-associated antigens; and
(c) comparing the measured amount of the anti-p53 antibody with a predetermined reference level of the anti-p53 antibody against the one or more tumor-associated antigens,

wherein the tumor-associated antigens include one or more proteins selected from the group consisting of wild-type p53 isoform A, p53 isoform B, p53 isoform C, and p53 isoform variants of Embodiment 1-1 and
an isotype of the anti-p53 antibody is one or more types selected from the group consisting of IgA, IgM, and IgE antibodies.

The wild-type p53 isoform A of Embodiment 1-3 may be a protein represented by any one of the following (i"') to (iii"').
(i"') A protein consisting of the amino acid sequence of SEQ ID NO: 1.
(ii‴) A protein consisting of an amino acid sequence having one or several amino acids added, deleted and/or substituted in the amino acid sequence of SEQ ID NO: 1 and capable of specifically binding to an anti-p53 antibody specifically binding to the protein of SEQ ID NO: 1 at a level equivalent to that for the protein of SEQ ID NO: 1.
(iii‴) A protein represented by any one of a protein consisting of an amino acid sequence having a homology of 95% or more to any one of the amino acid sequence of SEQ ID NO: 1 and capable of specifically binding to an anti-p53 antibody specifically binding to the protein of SEQ ID NO: 1 at a level equivalent to that for the protein of SEQ ID NO 1.

That is, the method of Embodiment 1-3, any one or more of the p53 isoforms A to C and the p53 isoform variants of Embodiment 1-1 are each used as a tumor-associated antigen, and the isotype of the antibody to be measured is one or more types selected from the group consisting of IgA, IgM, and IgE antibodies. Except for the above, the other configurations are the same as those of Embodiment 1-1 described above. Specifically, the definition of each term, the details of each step, the preferred embodiments, and the like are the same as those in Embodiment 1-1 described above.

For example, similar to Embodiment 1-1 described above, it is preferable to use two or more kinds among the wild-type p53 isoform A, the p53 isoform B, the p53 isoform C, and the p53 isoform variants of Embodiment 1-1 in combination as the tumor-associated antigens. Further, for example, the isotype of the anti-p53 antibody preferably includes two or more isotypes selected from the group consisting of IgA, IgM, and IgE antibodies. When the isotype of the anti-p53 antibody includes two or more isotypes, steps (b) to (c) are preferably performed using each isotype of the anti-p53 antibody. In addition, in the method of Embodiment 1-3, when two or more isotypes of the anti-p53 antibody are measured, the isotypes of the anti-p53 antibody may be a combination of an IgG antibody and one or more selected from the group consisting of IgA, IgM, and IgE antibodies.

### (Embodiment 1-4)

A method in Embodiment 1-4 is a method including measuring two or more isotypes of an anti-p53 antibody to collect data for diagnosing cancer, determining an onset risk of cancer, determining a malignancy grade of cancer, and/or predicting a prognosis of cancer.

As described in Embodiment 1-3, according to the findings found by the present inventors, it is assumed that the different kinds of the antibody isotype cause different cancer detection results by the amount of anti-p53 antibody that specifically binds to any one of proteins among p53 isoform A, p53 isoform B, p53 isoform C, and p53 isoform variants of Embodiment 1-1. Therefore, the method in Embodiment 1-4 described below allows for detection of a wide variety of the anti-p53 antibody in a sample, which antibody cannot be recognized by the conventional method. This thus makes it possible to collect data for diagnosing cancer, determining an onset risk of cancer, determining a malignancy grade of cancer, and/or predicting a prognosis of cancer with good sensitivity while maintaining high specificity.

A method in Embodiment 1-4 is a method of collecting data for diagnosing cancer, determining an onset risk of cancer, determining a malignancy grade of cancer, and/or predicting a prognosis of cancer, the method including the steps of:
(a) bringing a sample derived from a subject into contact with one or more tumor-associated antigens to cause an antigen-antibody reaction;
(b) measuring an amount of anti-p53 antibody, in the sample, that specifically binds to any of the one or more tumor-associated antigens; and
(c) comparing the measured amount of the anti-p53 antibody with a predetermined reference level of the anti-p53 antibody against the one or more tumor-associated antigens,

wherein the tumor-associated antigens include one or more proteins selected from the group consisting of wild-type p53 isoform A, p53 isoform B, p53 isoform C, and p53 isoform variants of Embodiment 1-1 and
an isotype of the anti-p53 antibody is two or more types selected from the group consisting of IgG, IgA, IgM, and IgE antibodies.

That is, in the method of Embodiment 1-4, any one or more of the p53 isoforms A to C and the p53 isoform variants of Embodiment 1-1 are each used as a tumor-associated antigen, and two or more antibody isotypes are measured. Except for the above, the other configurations are the same as those of Embodiment 1-1 described above. Specifically, the definition of each term, the details of each step, the preferred embodiments, and the like are the same as those in Embodiment 1-1 described above.

For example, similar to Embodiment 1-1 described above, it is preferable to use two or more kinds among the wild-type p53 isoform A, the p53 isoform B, the p53 isoform C, and the p53 isoform variants of Embodiment 1-1 in combination as the tumor-associated antigens. Further, for example, similar to Embodiment 1-1 described above, steps (b) to (c) are preferably performed using each isotype of the anti-p53 antibody. In addition, in the method of Embodiment 1-3, the isotype of the anti-p53 antibody preferably includes an IgG antibody and an IgA antibody, and is more preferably a combination of an IgG antibody and an IgA antibody.

### <Embodiment 2: Kit for diagnosing cancer>

The kit for diagnosing cancer in Embodiment 2 includes: a kit related to the above-described p53 isoform variants having a deletion mutation (Embodiment 2-1); a kit related to p53 isoform B, p53 isoform C, and the above-described p53 isoform variants having a deletion mutation (Embodiment 2-2); and a kit related to p53 isoform A, p53 isoform B, p53 isoform C, and the above-described p53 isoform variants having a deletion mutation (Embodiment 2-3). Note that the kit for diagnosing cancer in each Embodiment may optionally contain, for instance, any reagent and labeled antibody that are known to those skilled in the art and are necessary for measuring the amount of each anti-p53 antibody as a cancer marker. Details will be described below.

### (Embodiment 2-1)

The kit for diagnosing cancer in Embodiment 2-1 includes one or more of the above-described p53 isoform variants of Embodiment 1-1. The preferred amino acid sequence of each p53 isoform variant of Embodiment 1-1 as contained in the kit for diagnosing cancer is the same as the sequence described in Embodiment 1-1 mentioned above.

Similar to Embodiment 1-1 described above, the kit for diagnosing cancer may further include, in addition to the p53 isoform variants of Embodiment 1-1, any one or more of wild-type p53 isoform A, p53 isoform B, and p53 isoform C as the tumor-associated antigens. In addition, similar to Embodiment 1-1 described above, the kit for diagnosing cancer preferably contains two or more, more preferably three or more, still more preferably four or more, and particularly preferably five or more of the p53 isoform variants of Embodiment 1-1.

### (Embodiment 2-2)

The kit for diagnosing cancer in Embodiment 2-2 includes one or more of p53 isoform B, p53 isoform C, and the p53 isoform variants of Embodiment 1-1. The amino acid sequences of the p53 isoform B, the p53 isoform C, and the p53 isoform variants of Embodiment 1-1 as included in the kit for diagnosing cancer and preferred amino acid sequences thereof are substantially the same sequences described in Embodiment 1-1 and Embodiment 1-2 described above.

Similar to Embodiment 1-2 described above, the kit for diagnosing cancer in Embodiment 2-2 may include, as tumor-associated antigens, not only one or more of p53 isoform B, p53 isoform C, and the p53 isoform variants of Embodiment 1-1, but also wild-type p53 isoform A. In addition, similar to Embodiment 1-2 described above, the kit for diagnosing cancer preferably contains two or more, more preferably three or more, still more preferably four or more, and particularly preferably five or more of p53 isoform B, p53 isoform C, and the p53 isoform variants of Embodiment 1-1.

### (Embodiment 2-3)

The kit for diagnosing cancer in Embodiment 2-3 includes one or more of wild-type p53 isoform A, p53 isoform B, p53 isoform C, and the p53 isoform variants of Embodiment 1-1. The amino acid sequences of the wild-type p53 isoform A, the p53 isoform B, the p53 isoform C, and the p53 isoform variants of Embodiment 1-1 as included in the kit for diagnosing cancer and preferred amino acid sequences thereof are substantially the same sequences described in Embodiment 1-1, Embodiment 1-2, and Embodiment 1-3 described above.

In addition, similar to Embodiments 1-3 and 1-4 described above, the kit for diagnosing cancer in Embodiment 2-3 preferably contains two or more, more preferably three or more, still more preferably four or more, and particularly preferably five or more of wild-type p53 isoform A, p53 isoform B, p53 isoform C, and the p53 isoform variants of Embodiment 1-1.

The method and the kit for diagnosing cancer in Embodiments 1 and 2 of the present disclosure can substantially contribute to cancer diagnosis, onset risk determination of cancer, malignancy grade determination of cancer, and/or prognosis prediction of cancer. Furthermore, the present inventors have found the presence of anti-p53 antibodies having specific binding properties to p53 isoform variants or the like, which antibodies are not recognized by the wild-type p53 isoform A. This finding is expected to lead to, for instance, the development of antibodies and vaccines for various cancer treatments and therapies in the future.

Hereinabove, the outline of the present disclosure has been described. The following collectively provides a method of collecting data for diagnosing cancer, determining an onset risk of cancer, determining a malignancy grade of cancer, and/or predicting a prognosis of cancer and a kit for diagnosing cancer according to embodiments of the present disclosure.

A method according to a first aspect of the present disclosure is a method of collecting data for diagnosing cancer, determining an onset risk of cancer, determining a malignancy grade of cancer, and/or predicting a prognosis of cancer, the method including the steps of:
(a) bringing a sample derived from a subject into contact with one or more tumor-associated antigens to cause an antigen-antibody reaction;
(b) measuring an amount of anti-p53 antibody, in the sample, that specifically binds to any of the one or more tumor-associated antigens; and
(c) comparing the measured amount of the anti-p53 antibody with a predetermined reference level of the anti-p53 antibody against the one or more tumor-associated antigens,
wherein the tumor-associated antigens include one or more p53 isoform variants consisting of amino acid sequences having a mutation in an amino acid sequence of any one of SEQ ID NOs: 1 to 3, and the mutation includes any one of an N-terminal deletion mutation, a C-terminal deletion mutation, and both the N-terminal deletion mutation and the C-terminal deletion mutation.

In the above method, the p53 isoform variants are each preferably a protein represented by any one of
(i) a protein consisting of an amino acid sequence of any one of SEQ ID NOs: 4 to 35,
(ii) a protein consisting of an amino acid sequence having one or several amino acids added, deleted and/or substituted in the amino acid sequence of any one of SEQ ID NOs: 4 to 35 and capable of specifically binding to an anti-p53 antibody specifically binding to the protein of the corresponding SEQ ID NO described in (i) at a level equivalent to that for the protein of the corresponding SEQ ID NO, and
(iii) a protein consisting of an amino acid sequence having a homology of 95% or more to the amino acid sequence of any one of SEQ ID NOs: 4 to 35 and capable of specifically binding to an anti-p53 antibody specifically binding to the protein of the corresponding SEQ ID NO described in (i) at a level equivalent to that for the protein of the corresponding SEQ ID NO.

In the above method, the p53 isoform variants are each more preferably a protein represented by any one of
(I) a protein consisting of an amino acid sequence of any one of SEQ ID NOs: 4 to 6, 7, 8, 10, 11, 14, 16 to 24, 26, and 29 to 35,
(II) a protein consisting of an amino acid sequence having one or several amino acids added, deleted and/or substituted in the amino acid sequence of any one of SEQ ID NOs: 4 to 6, 7, 8, 10, 11, 14, 16 to 24, 26, and 29 to 35 and capable of specifically binding to an anti-p53 antibody specifically binding to the protein of the corresponding SEQ ID NO described in (I) at a level equivalent to that for the protein of the corresponding SEQ ID NO, and
(III) a protein consisting of an amino acid sequence having a homology of 95% or more to the amino acid sequence of any one of SEQ ID NOs: 4 to 6, 7, 8, 10, 11, 14, 16 to 24, 26, and 29 to 35 and capable of specifically binding to an anti-p53 antibody specifically binding to the protein of the corresponding SEQ ID NO described in (I) at a level equivalent to that for the protein of the corresponding SEQ ID NO.

In the above method, the tumor-associated antigens still more preferably further include one or more p53 isoforms each consisting of the amino acid sequence of any one of SEQ ID NOs: 1 to 3.

In the above method, it is particularly preferable that an isotype of the anti-p53 antibody is one or more types selected from the group consisting of IgG, IgA, IgM, and IgE antibodies, and
when there are two or more isotypes of the anti-p53 antibody, steps (b) to (c) are performed on each anti-p53 antibody isotype.

A kit for diagnosing cancer according to a second aspect of the present disclosure includes one or more p53 isoform variants consisting of amino acid sequences having a mutation in an amino acid sequence of any one of SEQ ID NOs: 1 to 3, wherein the mutation includes any one of an N-terminal deletion mutation, a C-terminal deletion mutation, and both the N-terminal deletion mutation and the C-terminal deletion mutation.

In the above kit for diagnosing cancer, the p53 isoform variants are each preferably a protein represented by any one of
(i) a protein consisting of an amino acid sequence of any one of SEQ ID NOs: 4 to 35,
(ii) a protein consisting of an amino acid sequence having one or several amino acids added, deleted and/or substituted in the amino acid sequence of any one of SEQ ID NOs: 4 to 35 and capable of specifically binding to an anti-p53 antibody specifically binding to the protein of the corresponding SEQ ID NO described in (i) at a level equivalent to that for the protein of the corresponding SEQ ID NO, and
(iii) a protein consisting of an amino acid sequence having a homology of 95% or more to the amino acid sequence of any one of SEQ ID NOs: 4 to 35 and capable of specifically binding to an anti-p53 antibody specifically binding to the protein of the corresponding SEQ ID NO described in (i) at a level equivalent to that for the protein of the corresponding SEQ ID NO.

In the above kit for diagnosing cancer, the p53 isoform variants are each more preferably a protein represented by any one of
(I) a protein consisting of an amino acid sequence of any one of SEQ ID NOs: 4 to 6, 7, 8, 10, 11, 14, 16 to 24, 26, and 29 to 35,
(II) a protein consisting of an amino acid sequence having one or several amino acids added, deleted and/or substituted in the amino acid sequence of any one of SEQ ID NOs: 4 to 6, 7, 8, 10, 11, 14, 16 to 24, 26, and 29 to 35 and capable of specifically binding to an anti-p53 antibody specifically binding to the protein of the corresponding SEQ ID NO described in (I) at a level equivalent to that for the protein of the corresponding SEQ ID NO, and
(III) a protein consisting of an amino acid sequence having a homology of 95% or more to the amino acid sequence of any one of SEQ ID NOs: 4 to 6, 7, 8, 10, 11, 14, 16 to 24, 26, and 29 to 35 and capable of specifically binding to an anti-p53 antibody specifically binding to the protein of the corresponding SEQ ID NO described in (I) at a level equivalent to that for the protein of the corresponding SEQ ID NO.

### Examples

Hereinafter, the present disclosure will be described more specifically with reference to Examples. The present disclosure, however, is not limited by the Examples at all.

In Examples, the presence of an anti-p53 antibody was examined, which antibody was specific for p53 isoform B, p53 isoform C, or each p53 isoform variant with any of deletion mutations including N-terminal deletion mutations, C-terminal deletion mutations, and both the N-terminal and C-terminal deletion mutations. Further, examined was whether or not there was a difference in the detection results between the IgG antibody and the IgA antibody.

Specifically, anti-p53 antibodies contained in a sample were profiled by IgG antibody or IgA antibody-mediated detection with a protein microarray using synthetic human proteins including wild-type p53 isoform A, p53 isoform B, p53 isoform C, and each p53 isoform variant. Here, 60 human plasma samples (sample Nos. 1 to 60) were used. A specific procedure will be described in detail below.

### <Example 1: Profiling of IgG antibodies specific for p53 isoform variants, p53 isoform B, or p53 isoform C>

In Example 1, among the 60 human plasma samples, positive samples were determined such that any of the anti-p53 IgG antibody for each p53 isoform variant, p53 isoform B, or p53 isoform C was detected in an amount equal to or greater than a reference level. As a control (Comparative Example), positive samples were determined such that the anti-p53 IgG antibody for the wild-type p53 isoform A was detected in an amount equal to or greater than a reference level.

### (Experimental procedure)

The protein microarray used was a microarray in which 16680 kinds of synthetic human proteins including wild-type p53 isoform A, p53 isoform B, p53 isoform C, and respective p53 isoform variants were arrayed. This protein microarray was produced by Fukushima Medical University Medical and Industrial Translational Research Center.

Wild-type p53 isoform A, p53 isoform B, and p53 isoform C are proteins consisting of the amino acid sequences represented by SEQ ID NOs: 1 to 3, respectively. The following p53 isoform variants were printed, including p53 isoforms having an N-terminal deletion mutation (p53 isoforms G, I, H, D, E, F, J, K, L), p53 isoforms having only a C-terminal deletion mutation due to a nonsense mutation, and p53 isoforms having both the N-terminal and C-terminal deletion mutations. Specifically, as shown in Tables 2 and 3 below, different p53 isoform variants were printed that consist of the amino acid sequences represented by SEQ ID NOs: 4 to 35 and have any one of deletion mutations including the N-terminal deletion mutations, the C-terminal deletion mutations, and both the N-terminal and C-terminal deletion mutations in the amino acid sequence of one of SEQ ID NOs: 1 to 3.

The protein microarray used was produced by the method described in Naoki Goshima, Yoshifumi Kawamura, Akiko Fukumoto et al., "Human protein factory for converting the transcriptome into an in vitro-expressed proteome.", Nature methods, 12, 1011-1027, 2008. Specifically, an array used as the protein microarray was produced by printing synthetic human proteins including wild-type p53 isoform A, p53 isoform B, p53 isoform C, and various p53 isoform variants on a slide glass (manufactured by Matsunami Glass Ind., Ltd.) according to the protocol recommended by the company while using an ultra-low-volume dispensing device (manufactured by Microdiagnostic Co., Ltd.).

The produced protein microarray was immersed in ethanol for 30 seconds, and the protein microarray was then washed twice for 30 seconds by using ultrapure water. Next, the protein microarray was immersed in dedicated protein microarray solution A (manufactured by Fukushima Protein Factory, Inc.) and shaken under an environment at 26°C for 5 minutes. Thereafter, the protein microarray was immersed in Blocking One (manufactured by NACALAI TESQUE, INC.), and shaken under an environment at 26°C for 1 hour. The solution was then replaced with dedicated protein microarray solution A (manufactured by Fukushima Protein Factory, Inc.).

Next, goat reference antibody mixture I (manufactured by Fukushima Protein Factory, Inc.) was added at 1:4000 to a protein microarray-dedicated primary antibody diluent (manufactured by Fukushima Protein Factory, Inc.). Further, each primary antibody solution was prepared by diluting 2000-fold, with the resulting solution, each of 60 human plasma samples. Here, 2 mL of each of the prepared primary antibody solutions was added to a protein microarray cassette P-DE1 (manufactured by Fukushima Protein Factory, Inc.). Subsequently, the protein microarray, in which the solution was replaced with solution A (manufactured by Fukushima Protein Factory, Inc.), was inserted into this cassette, and the cassette was capped. The capped protein microarray was then sealed using 8 dedicated clips and allowed to react in an environment at 37°C for 17 hours (primary antibody reaction).

After the primary antibody reaction, the protein microarray was washed 3 times for 1 minute in an environment at 26°C with dedicated protein microarray solution A (manufactured by Fukushima Protein Factory, Inc.). Thereafter, the washed protein microarray was allowed to stand for 5 minutes, and washed again 3 times for 3 minutes.

Next, a secondary antibody solution was prepared by adding Cy3-labeled anti-goat IgG antibody and Alexa Fluor647 (registered trademark)-labeled anti-human IgG antibody to a protein microarray-dedicated secondary antibody diluent (manufactured by Fukushima Protein Factory, Inc.) at 1:500. Here, 2 mL of the prepared secondary antibody solution was added to a protein microarray cassette P-DE1 (manufactured by Fukushima Protein Factory, Inc.). Thereafter, the washed protein microarray was inserted into the cassette and capped. The capped protein microarray was then sealed using 8 dedicated clips and allowed to react in an environment at 26°C for 1 hours (secondary antibody reaction).

After the secondary antibody reaction, the protein microarray was washed 3 times for 1 minute in an environment at 26°C with dedicated protein microarray solution A (manufactured by Fukushima Protein Factory, Inc.). Thereafter, the washed protein microarray was allowed to stand for 5 minutes, and washed again 3 times for 3 minutes.

Subsequently, the protein microarray was washed twice for 30 seconds with dedicated protein microarray solution B (manufactured by Fukushima Protein Factory, Inc.). Thereafter, the protein microarray was washed 4 times for 30 seconds with a protein microarray-dedicated final washing solution (manufactured by Fukushima Protein Factory, Inc.). The protein microarray after washing was centrifuged at 1500 rpm for 1 minute and dried. The protein microarray was then scanned using a GenePix 4000B scanner.

Numerical values between samples were normalized by the following method using Excel software (Microsoft, Bellevue, WA, USA). First, calculated was how many times the average value of all the measured values of the fluorescence intensity of Cy3 each numerical value of the fluorescence intensity of Cy3 derived from the reference antibody was. Next, the fluorescence intensity of Alexa Fluor^{®}647 at each protein spot for each sample as measured with a scanner was divided by the value obtained above. At that time, when the numerical value of the fluorescence intensity of Cy3 derived from the reference antibody was zero, the data of the spot was determined as spotting failure and was not used. Next, the background, which was the measured value of the spot to which no human plasma was added, was subtracted from the above divided value of the fluorescence intensity of the spot of each protein described above. Thereafter, the 25 percentile value for the respective samples was subtracted from the calculated value, and the resulting value was then divided by the SD value. The calculated value was then divided by the 75 percentile value for the respective samples, and the resulting value was multiplied by 10.

When the value thus normalized was more than 50, it was considered that the anti-p53 IgG antibody specific to the target protein (IgA antibody in Example 2 described later) was detected in an amount equal to or greater than the reference level. That is, the sample was determined to be positive by measuring the target protein. On the other hand, when the normalized value was 50 or less, the amount of the anti-p53 IgG antibody specific to the target protein (IgA antibody in Example 2 described later) was determined to be less than the reference level. That is, the sample was determined to be negative by measuring the target protein. Note that some of the proteins among wild-type p53 isoform A, p53 isoform B, p53 isoform C, and p53 isoform variants, which are target proteins, were each at a plurality of spots on one glass slide, and measured simultaneously. In this case, when half or more of the plurality of spots were determined to be positive, the protein was determined to be positive.

### (Results and discussion)

Table 2 below shows details of the structure of the amino acid sequence of each protein among wild-type p53 isoform A, p53 isoform B, p53 isoform C, and respective p53 isoform variants printed on the array, and details of the results of measuring the anti-p53 IgG antibodies against those proteins.

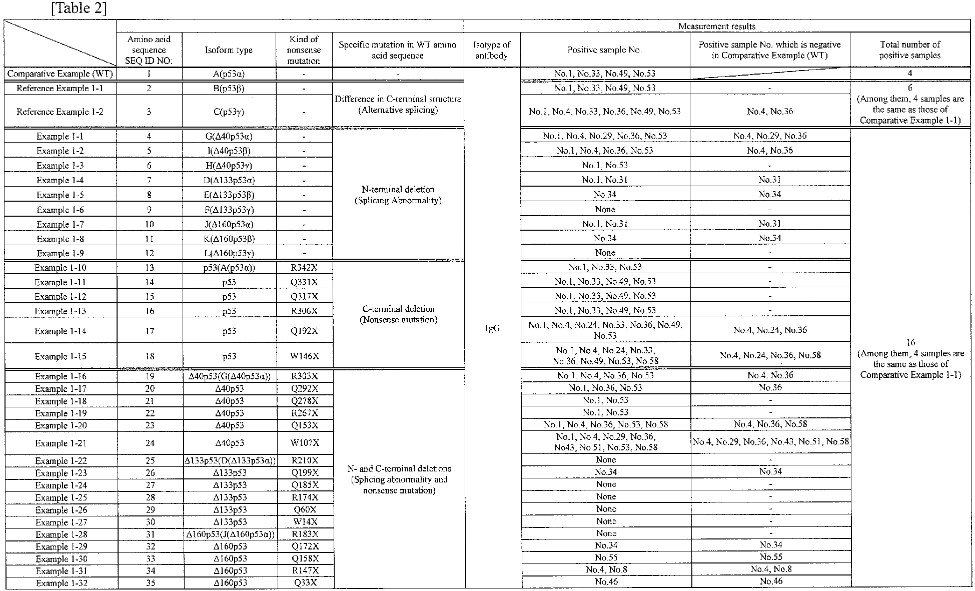

As shown in Table 2 above, the number of samples determined to be positive in the assay for the anti-p53 IgG antibody against wild-type isoform A of Comparative Example was 4 out of 60 samples.

By contrast, in the assay for the anti-p53 IgG antibody against each of the p53 isoform variants of Examples 1-1 to 1-32, only Examples 1-11, 1-12, and 1-13 had exactly the same results as the Comparative Example. In particular, in the assay for the anti-p53 IgG antibody against each of the p53 isoform variants of Examples 1-1, 1-2, 1-4, 1-5, 1-7, 1-8, 1-14 to 1-17, 1-20, 1-21, 1-23, and 1-29 to 1-32, samples determined to be negative in the Comparative Example were determined to be positive.

The results shown in Table 2 above have been found to demonstrate that there are a plurality of anti-p53 IgG antibodies that specifically bind to respective p53 isoform variants and cannot be recognized by the wild-type p53 isoform A.

Although not shown in Table 2 above, in the results of measuring an anti-p53 IgG antibody against the p53 variant having only an amino acid substitution caused by a missense mutation in the amino acid sequence represented by SEQ ID NO: 1, no significant difference was observed as compared with the Comparative Example. Therefore, it can be found that there are significantly more such specific anti-p53 antibodies against p53 isoform variants with any of an N-terminal deletion mutation, a C-terminal deletion mutation, and both the N-terminal and C-terminal deletion mutations.

In Examples 1-1 to 1-32, p53 isoform variants, for which the number of positive samples was zero, are also present. Nevertheless, based on various detection results of the positive samples in Example 1, it is assumed that an anti-p53 IgG antibody that specifically binds to each p53 isoform variant can be found by performing measurement on another sample.

These results have revealed that one or more p53 isoform variants of Examples 1-1 to 1-32 can be used in combination to detect an anti-p53 antibody that has not been conventionally recognized. By combining the detection results, it is sufficiently assumed that when data for diagnosing cancer or the like is collected, sensitivity can be improved while high specificity is maintained.

Further, as shown in Table 2 above, in the assay for the anti-p53 IgG antibody against p53 isoform C of Reference Example 1-2, the samples determined to be negative in the Comparative Example were determined to be positive.

The results of Reference Example 1-2 shown in Table 2 above have revealed the presence of an anti-p53 IgG antibody that specifically binds to the p53 isoform C and cannot be recognized by the wild-type isoform A.

Note that the p53 isoform B assay results in Reference Example 1-1 were the same as those of the Comparative Example (WT). However, based on various detection results of the positive samples in Reference Example 1-2 and Examples 1-1 to 1-32, it is assumed that an anti-p53 IgG antibody that specifically binds to the p53 isoform B can be found by performing measurement on another sample.

These results have demonstrated that one or more p53 isoform variants among p53 isoform B, p53 isoform C, and Examples 1-1 to 1-32 can be used in combination to detect an anti-p53 antibody that has not been conventionally recognized. As a result, it is sufficiently assumed that when data for diagnosing cancer or the like is collected, sensitivity can be improved while high specificity is maintained.

### <Example 2: Profiling of IgA antibodies specific for p53 isoform variants or p53 isoforms A to C>

In Example 2, among the 60 human plasma samples, positive samples were determined such that any of the anti-p53 IgA antibody for each p53 isoform variant, or p53 isoforms A to C was detected in an amount equal to or greater than a reference level. As a control (Comparative Example), positive samples were set such that the anti-p53 IgG antibody for the wild-type p53 isoform A was detected in an amount equal to or greater than a reference level in the same manner as in Example 1.

### (Experimental procedure)

A primary antibody solution was prepared by adding an antibody obtained beforehand by reacting a goat reference antibody mixture II and a Cy3-labeled anti-goat IgG antibody to a protein microarray-dedicated primary antibody diluent at 1:4000. A secondary antibody solution was prepared by adding a DyLight650-labeled anti-human IgA antibody to a protein microarray-dedicated secondary antibody diluent at 1:500. Except for the above, the same procedure as in Example 1 was repeated for assay, normalization, and positive or negative determination for the anti-p53 IgA antibody against each p53 isoform variant or p53 isoforms A to C.

### (Results and discussion)

Table 3 below shows details of the structure of the amino acid sequence of each protein among wild-type p53 isoform A, p53 isoform B, p53 isoform C, and respective p53 isoform variants printed on the array, and details of the results of measuring the anti-p53 IgG or IgA antibodies against those proteins.

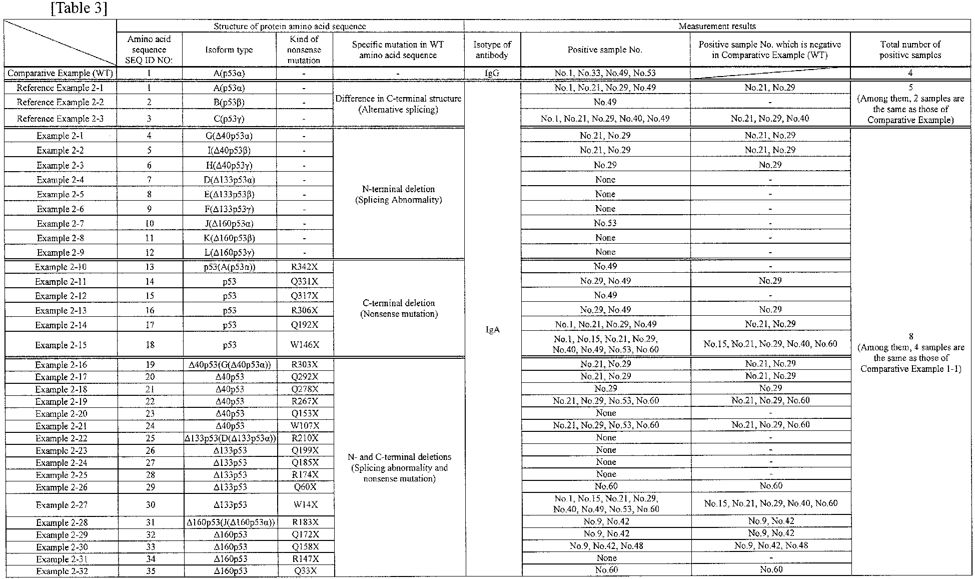

As described above, the number of samples determined to be positive in the assay for the anti-p53 IgG antibody against wild-type isoform A of Comparative Example was 4 out of 60 samples. By contrast, as shown in Table 3 above, in the assay for the anti-p53 IgA antibody against each of the p53 isoform variants of Examples 2-1 to 2-32, there was no Example having the same results as those of the Comparative Example. In particular, in the assay for the anti-p53 IgA antibody against each of the p53 isoform variants of Examples 2-1 to 2-3, 2-11, 2-13 to 2-19, 2-21, 2-26 to 2-30, and 2-32, samples determined to be negative in the Comparative Example were determined to be positive.

The results shown in Table 3 above have been found to demonstrate that there are a plurality of not only the IgG antibodies but also anti-p53 IgA antibodies that specifically bind to respective p53 isoform variants. In addition, by comparing Table 1 in Example 1 with Table 2 described above, even when the same p53 isoform variants were used, it is found that the results of detecting the positive samples by the IgG antibodies do not necessarily coincide with the results of detecting the positive samples by the IgA antibodies.

In Examples 2-1 to 2-32, p53 isoform variants, for which the number of positive samples was zero, are also present. Nevertheless, based on various detection results of the positive samples in Example 2, it is assumed that an anti-p53 IgA antibody that specifically binds to each p53 isoform variant can be found by performing measurement on another sample.

These results have demonstrated that by measuring both the IgG antibodies and IgA antibodies while using a combination of one or more p53 isoform variants of Examples 2-1 to 2-32 (p53 isoform variants represented by the amino acid sequences of SEQ ID NOs: 4 to 35), a wide variety of anti-p53 antibody that has not been conventionally recognized can be detected. Then, by combining the results, it is sufficiently assumed that when data for diagnosing cancer or the like is collected, sensitivity can be improved more while high specificity is maintained.

Further, as shown in Table 3 above, in Reference Examples 2-1 to 2-3, there was no Reference Example having the same result as that of the Comparative Example. In particular, in the assay for the anti-p53 IgA antibody against wild-type p53 isoform A of Reference Example 2-1 or p53 isoform C of Reference Example 2-3, a total of three samples determined to be negative in the Comparative Example were determined to be positive.

The results shown in Table 3 above have been found to demonstrate that there are a plurality of not only the IgG antibodies but also anti-p53 IgA antibodies that specifically bind to some of p53 isoforms A to C. In addition, by comparing the Comparative Example and the Reference Example in Table 2 of Example 1 with the Reference Example in Table 3 above, even when the same p53 isoforms A to C were used, it is found that the results of detecting the positive samples by the IgG antibodies do not necessarily coincide with the results of detecting the positive samples by the IgA antibodies.

In Reference Example 2-2, no positive sample other than the positive samples determined to be positive in the Comparative Example was detected. However, based on various detection results of the positive samples in Reference Example 2-1 and 2-3 and Examples 2-1 to 2-32, it is assumed that an anti-p53 IgA antibody that specifically binds to the p53 isoform B can be found by performing measurement on another sample.

These results have demonstrated that by measuring both the IgG antibodies and IgA antibodies while using a combination of one or more p53 isoform variants of Examples 2-1 to 2-32 and p53 isoforms A to C, a wider variety of anti-p53 antibody that has not been conventionally recognized can be detected. As a result, it is sufficiently assumed that when data for diagnosing cancer or the like is collected, sensitivity can be improved more while high specificity is maintained.

For example, Table 4 below shows the detection results of positive samples by the p53 isoform variants of Examples 1-1 to 1-32 and Examples 2-1 to 2-32 related to the method in this Embodiment 1-1.

**[Table 4]**

| | Comparative Example | Example 1-1 to Example 1-32 and Example 2-1 to Example 2-32 |
|---|---|---|
| Positive sample No. | No.1, No.33, No.49, No.53 | No.1, No.4, No.8, No.9, No.15, No.21, No.24, No.29, No.31, No.33, No.34, No.36, No.40, No.42, No.43, No.46, No.48, No.49, No.51, No.53, No.55, No.58, No.60 |
| Total number of positive samples | 4 | 23 (Among them, 4 samples are the same as those of Comparative Example) |

As shown in Table 4 above, when both the anti-p53 IgG antibodies and anti-p53 IgA antibodies are measured using all of the p53 isoform variants represented by the amino acid sequences of SEQ ID NOs: 4 to 35, 23 positive samples among 60 samples are detected. On the other hand, according to the conventional method of measuring the anti-p53 IgG antibody while using the wild-type isoform A, only 4 positive samples are detected. That is, it is assumed that there are a large number of samples that are actually positive but determined as negative in the conventional method.

As such, according to the results of Examples 1 and 2, by measuring both the IgG antibodies and IgA antibodies while using a combination of one or more p53 isoforms A to C and p53 isoform variants represented by the amino acid sequences of SEQ ID NOs: 1 to 35, a wide variety of anti-p53 antibody that has not been conventionally recognized can be detected. As a result, it is assumed that when data for diagnosing cancer or the like is collected, sensitivity can be improved markedly while high specificity is maintained.

The present application is based on Japanese Patent Application No. 2022-144861 filed on September 12, 2022, the contents of which are included in the present application.

In order to express the present disclosure, the present invention has been appropriately and sufficiently described through the Embodiments and Examples with reference to, for instance, the above-described specific examples. However, it should be recognized that those skilled in the art can easily modify and/or improve the above-described Embodiments and Examples. Therefore, unless a modification or improvement made by a person skilled in the art is at a level that departs from the scope of the claims described in the Claims, the modification or improvement is interpreted to be included in the scope of the Claims.

### Industrial Applicability

The method and the kit for diagnosing cancer in the present disclosure can substantially contribute to cancer diagnosis, onset risk determination, malignancy grade determination, and/or prognosis prediction. Furthermore, the present inventors have found the presence of anti-p53 antibodies having specific binding properties to p53 isoform variants or the like, which antibodies are not recognized by the wild-type p53 isoform A. This finding is expected to lead to, for instance, the development of antibodies and vaccines for various cancer treatments and therapies in the future.

## Claims

1. A method of collecting data for diagnosing cancer, determining an onset risk of cancer, determining a malignancy grade of cancer, and/or predicting a prognosis of cancer, the method comprising the steps of:
(a) bringing a sample derived from a subject into contact with one or more tumor-associated antigens to cause an antigen-antibody reaction;
(b) measuring an amount of anti-p53 antibody, in the sample, that specifically binds to any of the one or more tumor-associated antigens; and
(c) comparing the measured amount of the anti-p53 antibody with a predetermined reference level of the anti-p53 antibody against the one or more tumor-associated antigens,
wherein the tumor-associated antigens include one or more p53 isoform variants consisting of amino acid sequences having a mutation in an amino acid sequence of any one of SEQ ID NOs: 1 to 3, and the mutation includes any one of an N-terminal deletion mutation, a C-terminal deletion mutation, and both the N-terminal deletion mutation and the C-terminal deletion mutation.

2. The method according to claim 1, wherein
the p53 isoform variants are each a protein represented by any one of
(i) a protein consisting of an amino acid sequence of any one of SEQ ID NOs: 4 to 35,
(ii) a protein consisting of an amino acid sequence having one or several amino acids added, deleted and/or substituted in the amino acid sequence of any one of SEQ ID NOs: 4 to 35 and capable of specifically binding to an anti-p53 antibody specifically binding to the protein of the corresponding SEQ ID NO described in (i) at a level equivalent to that for the protein of the corresponding SEQ ID NO, and
(iii) a protein consisting of an amino acid sequence having a homology of 95% or more to the amino acid sequence of any one of SEQ ID NOs: 4 to 35 and capable of specifically binding to an anti-p53 antibody specifically binding to the protein of the corresponding SEQ ID NO described in (i) at a level equivalent to that for the protein of the corresponding SEQ ID NO.

3. The method according to claim 1, wherein
the p53 isoform variants are each a protein represented by any one of
(I) a protein consisting of an amino acid sequence of any one of SEQ ID NOs: 4 to 6, 7, 8, 10, 11, 14, 16 to 24, 26, and 29 to 35,
(II) a protein consisting of an amino acid sequence having one or several amino acids added, deleted and/or substituted in the amino acid sequence of any one of SEQ ID NOs: 4 to 6, 7, 8, 10, 11, 14, 16 to 24, 26, and 29 to 35 and capable of specifically binding to an anti-p53 antibody specifically binding to the protein of the corresponding SEQ ID NO described in (I) at a level equivalent to that for the protein of the corresponding SEQ ID NO, and
(III) a protein consisting of an amino acid sequence having a homology of 95% or more to the amino acid sequence of any one of SEQ ID NOs: 4 to 6, 7, 8, 10, 11, 14, 16 to 24, 26, and 29 to 35 and capable of specifically binding to an anti-p53 antibody specifically binding to the protein of the corresponding SEQ ID NO. described in (I) at a level equivalent to that for the protein of the corresponding SEQ ID NO.

4. The method according to any one of claims 1 to 3, wherein the tumor-associated antigens further include one or more p53 isoforms each consisting of an amino acid sequence of any one of SEQ ID NOs: 1 to 3.

5. The method according to any one of claims 1 to 3, wherein
an isotype of the anti-p53 antibody is one or more types selected from the group consisting of IgG, IgA, IgM, and IgE antibodies, and
when there are two or more isotypes of the anti-p53 antibody, steps (b) to (c) are performed on each anti-p53 antibody isotype.

6. kit for diagnosing cancer, comprising one or more p53 isoform variants consisting of amino acid sequences having a mutation in an amino acid sequence of any one of SEQ ID NOs: 1 to 3,
wherein the mutation includes any one of an N-terminal deletion mutation, a C-terminal deletion mutation, and both the N-terminal deletion mutation and the C-terminal deletion mutation.

7. The kit for diagnosing cancer according to claim 6, wherein
the p53 isoform variants are each a protein represented by any one of
(i) a protein consisting of an amino acid sequence of any one of SEQ ID NOs: 4 to 35,
(ii) a protein consisting of an amino acid sequence having one or several amino acids added, deleted and/or substituted in the amino acid sequence of any one of SEQ ID NOs: 4 to 35 and capable of specifically binding to an anti-p53 antibody specifically binding to the protein of the corresponding SEQ ID NO described in (i) at a level equivalent to that for the protein of the corresponding SEQ ID NO, and
(iii) a protein consisting of an amino acid sequence having a homology of 95% or more to the amino acid sequence of any one of SEQ ID NOs: 4 to 35 and capable of specifically binding to an anti-p53 antibody specifically binding to the protein of the corresponding SEQ ID NO described in (i) at a level equivalent to that for the protein of the corresponding SEQ ID NO.

8. The kit for diagnosing cancer according to claim 6 or 7, wherein
the p53 isoform variants are each a protein represented by any one of
(I) a protein consisting of an amino acid sequence of any one of SEQ ID NOs: 4 to 6, 7, 8, 10, 11, 14, 16 to 24, 26, and 29 to 35,
(II) a protein consisting of an amino acid sequence having one or several amino acids added, deleted and/or substituted in the amino acid sequence of any one of SEQ ID NOs: 4 to 6, 7, 8, 10, 11, 14, 16 to 24, 26, and 29 to 35 and capable of specifically binding to an anti-p53 antibody specifically binding to the protein of the corresponding SEQ ID NO described in (I) at a level equivalent to that for the protein of the corresponding SEQ ID NO, and
(III) a protein consisting of an amino acid sequence having a homology of 95% or more to the amino acid sequence of any one of SEQ ID NOs: 4 to 6, 7, 8, 10, 11, 14, 16 to 24, 26, and 29 to 35 and capable of specifically binding to an anti-p53 antibody specifically binding to the protein of the corresponding SEQ ID NO described in (I) at a level equivalent to that for the protein of the corresponding SEQ ID NO.
